(19) **Europäisches Patentamt / European Patent Office / Office européen des brevets**

(11) **EP 1 786 316 B1**

(12) **EUROPEAN PATENT SPECIFICATION**

(45) Date of publication and mention of the grant of the patent:
**24.10.2012 Bulletin 2012/43**

(21) Application number: **05752203.9**

(22) Date of filing: **15.06.2005**

(51) Int Cl.:
*A61B 5/026* (2006.01)    *A61B 5/053* (2006.01)
*A61B 5/04* (2006.01)

(86) International application number:
**PCT/IL2005/000632**

(87) International publication number:
**WO 2006/011128 (02.02.2006 Gazette 2006/05)**

(54) **CEREBRAL PERFUSION MONITOR**

HIRNPERFUSIONSMONITOR

APPAREIL DE SURVEILLANCE DE PERFUSION CEREBRALE

(84) Designated Contracting States:
**AT BE BG CH CY CZ DE DK EE ES FI FR GB GR HU IE IS IT LI LT LU MC NL PL PT RO SE SI SK TR**

(30) Priority: **15.07.2004 US 893570**

(43) Date of publication of application:
**23.05.2007 Bulletin 2007/21**

(73) Proprietor: **Orsan Medical Technologies Ltd.**
**42504 Netanya (IL)**

(72) Inventors:
• **SHAPIRA, Aharon**
**43905 MOSHAV GIVAT CHEN (IL)**
• **RAPPAPORT, Alon**
**69407 TEL-AVIV (IL)**
• **BEN-ARI, Shlomi**
**30500 Binyamina (IL)**
• **REICHMAN, Yosef**
**44201 Kfar-Saba (IL)**

• **BARNEA, Ofer**
**46245 Herzliyah (IL)**

(74) Representative: **Machtalère, Georges et al**
**Dennemeyer & Associates S.A.**
**55, rue des Bruyères**
**1274 Howald (LU)**

(56) References cited:
**WO-A-03/059164     US-A1- 2004 034 294**
**US-B1- 6 223 069**

• JEVNING ET AL: "Evaluation of consistency among different electrical impedance indices of relative cerebral blood flow in normal resting individuals" JOURNAL OF BIOMEDICAL ENGINEERING, BUTTERWORTH, GUILDFORD, GB, vol. 11, no. 1, 1 January 1989 (1989-01-01), pages 53-56, XP022444925 ISSN: 0141-5425

**Description**

RELATED APPLICATIONS

The present application is a continuation-in-part of US patent application 10/893,570, filed July 15, 2004, which is a continuation-in-part of PCT patent application PCT/IL03/00042, filed January 15, 2003, which claims benefit under 35 USC 119(e) from US provisional patent application 60/348,278, filed January 15, 2002. This application is related to a PCT application filed on even date, titled "Device For Monitoring Blood Flow To Brain," having attorney docket, number 371/04609.

FIELD OF THE INVENTION

The field of the invention relates to measuring blood flow in the head.

BACKGROUND OF THE INVENTION

There is a need to measure cerebral blood flow during various medical events and procedures, because any disturbance to the flow of blood to the brain may cause injury to the function of the brain cells, and even death of brain cells If the disturbance is prolonged. Maintaining blood flow to the brain is especially important because brain cells are more vulnerable to a lack of oxygen than other cells, and because brain cells usually cannot regenerate following an injury.

A number of common situations may cause a decrease in the general blood flow to the brain, including arrhythmia, myocardial infarction, and traumatic hemorrhagic shock, A sudden increase in blood flow to the brain may also cause severe damage, and is especially likely to occur in newborn or premature babies, although such an increase may also occur in other patients with certain medical conditions, or during surgery. In all these cases, data regarding the quantity of blood flow in the brain, and the changes in flow rate, may be important in evaluating the risk of injury to the brain tissue and the efficacy of treatment. The availability of such data may enable the timely performance of various medical procedures to increase, decrease, or stabilize the cerebral blood flow, and prevent permanent damage to the brain.

In the absence of a simple means for direct and continuous monitoring of cerebral blood flow, information about changes in cerebral blood flow is inferred indirectly by monitoring clinical parameters which can be easily measured, such as blood pressure. But due to the different relation between blood pressure and cerebral blood flow in different medical conditions, there may be situations in which cerebral blood flow is inadequate even when blood pressure appears to be adequate. Cerebral blood flow may also be inferred indirectly by monitoring neurological function, but since neurological dysfunction is often irreversible by the time it is detected, it is more desirable to detect changes in cerebral blood flow directly, while its effects on brain function are still reversible.

Existing means for measuring cerebral blood flow are complex, expensive, and in some cases invasive, which limits their usefulness. Three methods that are presently used only in research are 1) injecting radioactive xenon into the cervical carotid arteries and observing the radiation it emits as it spreads throughout the brain; 2) positron emission tomography, also based on the injection of radioactive material; and 3) magnetic resonance angiography, performed using a room-sized, expensive, magnetic resonance imaging system, and requiring several minutes to give results. These three methods can only be carried out in a hospital or other center that has the specialized equipment available, and even in a hospital setting it is not practical to monitor patients continuously using these methods.

A fourth method, trans-cranial Doppler (TCD) uses ultrasound, is not invasive and gives immediate results. However, TCD fails to give a correct determination of blood flow in about 15% of patients, due to the difficulty of passing sound waves through the cranium, and it requires great skill by professionals who have undergone prolonged training and practice in performing the test and deciphering the results. Another disadvantage of TCD is that it measures only regional blood flow in the brain, and does not measure global blood flow. Doppler ultrasound may also be used to measure blood flow in the carotid arteries, providing an estimate of blood flow to the head, but not specifically to the brain, and not including blood flow to the head through the vertebral arteries, which is difficult to measure by ultrasound because of their proximity to the vertebrae.

Two additional techniques that are used, generally in research, to measure blood flow in the head and in other parts of the body are electric impedance plethysmography (IPG) and photoplethysmography (PPG). US patent 6,819,950, to Mills, describes the use of PPG to detect carotid stenosis, among other conditions. US patent 5,694,939, to Cowings, describes biofeedback techniques for controlling blood pressure, which include the use of IPG in the lower leg and PPG in the finger. US patent 5,396,893, to Oberg et al, states that PPG is superior to IPG for monitoring patients' cardiac and respiration rates. US patent 6,832,113, to Belalcazar, describes the use of either PPG or PPG to measure blood flow, for purposes of optimizing a cardiac pacemaker. US patent 6,169,914, to Holland et al, describes the use of various types of sensors, including IPG and PPG, for monitoring female sexual arousal with a vaginal probe, and describes using different types of sensors in combination.

US patent 6,413,223, to Yang et al, describes a probe, used, on the finger, which contains two PPG sensors and one IPG sensors. The combined data from the three sensors, analyzed using a mathematical model of arterial blood flow, provides a more accurate measurement of blood flow than would be obtained by using IPG or IPG alone.

J. H. Seipel and J. E. Floam, in J. Clinical Pharmacology 15, 144-154 (1975) present the results of a clinical study of the effects of a drug, betahistidine, on cerebral, cranial, scalp and calf blood circulation. Rheoencepbalography (REG), a form of IPG, was used to measure the amplitude of cerebral blood flow.

R. Jevning, G. Fernando, and A. F. Wilson, "Evaluation of consistency among different electrical impedance indices of relative cerebral blood flow in normal resting individuals," J. Biomed Eng. 11, pages 53-56 (1989), describes measures derived from the impedance wave form Z(t) that have been found to correlate with regional cerebral blood flow. The measures include the amplitude $\Delta Z$, the time to peak T, the maximum rate of change dZ/dt, and measures involving the curvature of Z(t).

Published patent application US 2004/0034294 A1 to Kimball et al, describes a pulse oximeter system which can be mounted on a patient's finger, or another part of the body such as the head, using LEDs at different wavelengths, to measure the level of oxygenated and de-oxgenated hemoglobin the blood, as they change over a pulse cycle. To reduce motion-induced errors in this data, the pulse-oximeter may be triggered by, or its output analyzed using, data on a pulsatile characteristic of the patient, such as impedance data. A Z-enhanced pulse-oximeter is described that includes both an impedance measuring unit and a pulse-oximeter module incorporated into the same housing.

SUMMARY OF THE INVENTION

An aspect of some embodiments of the invention relates to estimating cerebral blood flow, by 1) using IPG to obtain a measure of the combined change in cerebral and including scalp blood volume during a cardiac cycle; 2) using PPG or another method, including surface IPG or ultrasonics, to obtain a measure of the change mainly in scalp blood volume; and 3) combining the two measurements to find the change in cerebral blood volume. The cerebral blood flow is then optionally found from the time derivative of the cerebral blood volume. Since there is generally a component of cerebral blood flow that is not associated with varying cerebral blood volume, in addition to a component associated with the variation in cerebral blood volume over a cardiac cycle, using the time derivative of the cerebral blood volume may only give an indication of the relative cerebral blood flow, rather than the absolute cerebral blood flow.

Optionally, the time-varying part of the cerebral blood volume is found by subtracting a weighted or normalized PPG signal from the IPG signal, to obtain a measure that depends primarily on the time-varying part of the cerebral blood volume, with relatively little dependence on the time-varying part of the scalp blood volume. Optionally, the weighting factor is estimated by using the fact that there is a time delay between the cerebral blood flow and the scalp blood flow, in each cardiac cycle, and assuming that in a later part of each cardiac cycle, for example the last third of each cycle, when the blood pressure is decreasing, the IPG signal is dominated by the time-varying part of the scalp blood volume. Alternatively or additionally, the weighting factor is estimated by using the power spectra and cross-power spectrum of the IPG and PPG signals. For example, the cross-power spectrum is used to find a range of frequencies for which the IPG and PPG signals are similar, and the weighting factor is set equal to the square root of the ratio between the power spectrum of the IPG signal integrated over those frequencies, and the power spectrum of the PPG signal integrated over those frequencies.

Optionally, the IPG measurement is made by placing IPG electrode units on two sides of the head, for example on the left and right temples. Optionally, one or both of the IPG electrode units is combined with a PPG sensor, in a single unit. Optionally, the IPG electrode units include separate current-carrying and voltage-measuring electrodes. For example, the current-carrying electrode may be in the form of a concentric ring surrounding the voltage-measuring electrode, or vice versa.

Some embodiments described relates to estimating cerebral blood flow by using characteristics of the IPG signal alone but do not form part of the invention . For example, the cerebral blood flow is estimated from the peak value of the IPG signal in each cardiac cycle, or from the peak rate of rise of the IPG signal after the beginning of each cardiac cycle, or from the height of the first local peak or inflection point in the IPG signal after the beginning of each cardiac cycle. The beginning of each cardiac cycle is defined, for example, by the peak of the R-wave of an ECG, or by the time of the minimum in the IPG or PPG signal, or by the time of the diastolic pressure. The rapid initial rate of rise in the IPG signal, up to the peak or up to the first local peak or inflection point, may be dominated by the cerebral blood flow, even if the IPG signal during the rest of the cardiac cycle is largely influenced by the scalp blood volume, since the scalp blood volume, as indicated by PPG data, generally rises more slowly, and with a delay, at the beginning of each cardiac cycle. Optionally, PPG data is also obtained, to confirm that the scalp blood volume is rising slowly initially, and that the rapid initial rise of the IPG signal is indeed due mostly to the cerebral blood flow.

Some embodiments of the invention may be particularly useful for monitoring premature infants, for example those with weight under 1.5 kg, who generally have poor ability to maintain constant blood flow to the brain due to the immaturity of their cerebral blood flow autoregulation system. Abrupt changes in blood flow to the brain can be caused by changes in respiration, changes in blood pressure, and manipulation of the infants by medical staff. Such abrupt changes in cerebral blood flow, if not immediately detected and treated, can cause severe brain injury, including injuries caused by cerebral hemorrhage which occurs in 10% to 30% of premature babies. The invention may also be useful in monitoring mature babies who may be at risk of brain hemorrhage or ischemia for various reasons.

The invention may also be useful for monitoring cerebral blood flow in 1) patients undergoing surgery of the carotid arteries, in which a clamp is applied to one of the carotids, potentially reducing blood flow to the brain; 2) patients with stenosis or occlusion of the carotid arteries or cerebral arteries, particularly if they are undergoing procedures such as intra-arterial catherization or stent application in the affected arteries; 3) brain injury patients, in whom brain edema might cause a decrease in blood perfusion, and herniation of the brain; 4) neurosurgery patients, during and for a few days after the surgery, when cerebral blood flow is often impaired; 5) patients undergoing other major surgery, including heart surgery, in which massive bleeding and resulting hypotension could lead to a decrease in cerebral blood flow. In all of these categories of patients, monitoring of cerebral blood flow could lead to prompt intervention before brain injury occurs.

An aspect of some embodiments of the invention relates to a probe including both electrical and scalp blood flow measurement sensors. Optionally, the probe is configured so that when placed at a certain (optionally pre-determined) location on the skull, for example, the temple, the blood flow measurement probe will be aimed at the vascular bed (e.g., source) of the location where electric field will be sensed.

There is thus provided in accordance with an exemplary embodiment of the invention, a method of estimating cerebral blood flow, comprising:

a) obtaining a measure of time-varying blood volume in the head, using impedance plethysmography;
b) obtaining a measure of time-varying blood volume in the scalp; and
c) using the measure of time-varying blood volume in the head and time-varying blood volume in the scalp to estimate the cerebral blood flow.

Optionally, obtaining a measure of time-varying blood flow in the scalp comprises using photoplethysmography

In an exemplary embodiment of the invention, estimating the cerebral blood flow comprises estimating the relative cerebral blood flow as it changes over time.

In an exemplary embodiment of the invention, using the measures of time-varying blood volume comprises finding a difference between weighted measures of time-varying blood volume.

In an exemplary embodiment of the invention, the measures of time-varying blood volume are weighted to have at least approximately the same value at a time in the cardiac cycle when the blood pressure is falling.

In an exemplary embodiment of the invention, the measures of time-varying blood volume are weighted to have approximately equal power spectra at frequencies for which the cross-power spectrum between the measures of time-varying blood volume is relatively high.

In an exemplary embodiment of the invention, obtaining a measure of blood volume in the head using impedance plethysmography comprises:

a) passing a current through the head using two current-carrying electrodes; and
b) measuring a voltage across the head, associated with the current, using two voltage-measuring electrodes.

Optionally, the method includes applying to the head an annular electrode surrounding at least one of the current-carrying electrodes, and maintaining the annular electrode at a same voltage as the current-carrying electrode it surrounds, thereby suppressing radial current from said current-carrying electrode.

Alternatively or additionally, the voltage-measuring electrodes are distinct from, and substantially electrically decoupled from, the current-carrying electrodes.

In an exemplary embodiment of the invention, obtaining a measure of blood volume in the head using impedance plethysmography comprises placing the two current-carrying electrodes on the left and right temples respectively.

In an exemplary embodiment of the invention, obtaining a measure of blood volume in the head using impedance plethysmography comprises placing each of the two voltage-measuring electrodes on the head in a position adjacent to a different one of the current-carrying electrodes. Optionally, obtaining a measure of blood volume in the scalp using photoplethysmography comprises placing a photoplethysmography sensor on the head adjacent to one of the current-carrying electrodes and to the voltage-measuring electrode which is adjacent to said current-carrying electrode.

There is also provided in accordance with an exemplary embodiment of the invention, a method of estimating cerebral blood flow, comprising:

a) measuring an impedance across the head as a function of time in a cardiac cycle; and
b) estimating the cerebral blood flow from a rate of change of the impedance during a time in the cardiac cycle when the blood pressure is rising.

There is also provided in accordance with an exemplary embodiment of the invention, a unit for estimating cerebral blood flow, adapted for placing on the head, the unit comprising:

a) at least one electrode adapted for impedance plethysmography; and
b) a plethysmography sensor adapted for measuring blood flow in a scalp.

Optionally, the senor is a photoplethysmography sensor.

In an exemplary embodiment of the invention, the unit comprises a signal processor configured to process one or both of data from the photoplethysmography sensor and impedance plethysomography data from the electrode.

In an exemplary embodiment of the invention, the at least one electrodes comprise:

a) a current-carrying electrode adapted for injecting current through the head when it is placed on the skin; and

b) a voltage-measuring electrode adapted for measuring voltage across the head when it is placed on the skin, and when the current-carrying electrode is injecting current.

In an exemplary embodiment of the invention, the current-carrying and voltage-measuring electrodes are configured such that the voltage measuring electrode will measure a potential substantially equal to a potential at the dermis, largely excluding the voltage drop across the epidermis, when the current-carrying electrode is injecting current.

In an exemplary embodiment of the invention, the unit is adapted for use in patients of a range of degree of maturity, wherein the current-carrying electrode comprises an annulus surrounding the voltage-measuring electrode, and the radial thickness of the annulus and the gap between the current-carrying and voltage-measuring electrodes are each at least twice as great as a typical thickness of the epidermis in patients of said range of degree of maturity.

In an exemplary embodiment of the invention, the radial thickness of the annulus and the gap between the current-carrying and voltage-measuring electrodes are each at least 1mm.

In an exemplary embodiment of the invention, the radial thickness of the annulus and the gap between the current-carrying and voltage-measuring electrodes are each at least 2 mm.

In an exemplary embodiment of the invention, the unit includes an annular electrode surrounding the current-carrying electrode, thereby suppressing radial current from the current-carrying electrode when the annular electrode is maintained at the same voltage as the current-carrying electrode.

There is also provided in accordance with an exemplary embodiment of the invention, a system for estimating cerebral blood flow, comprising:

a) at least one unit as described herein;

b) an impedance measuring unit comprising at least one electrode adapted for placing on the head and performing impedance plethysmography;

c) a power supply adapted for passing current across the head between one of the at least one electrodes of the one unit and one of the at least one electrodes of the impedance measuring unit, when said units are placed on different sides of the head; and

d) a data analyzer which calculates a cerebral blood flow using impedance data obtained from a voltage difference measured between one of the at least one electrodes of the one unit and one of the at least one electrodes of the impedance measuring unit, and from photoplethysmography data generated by the photoplethysmography sensor.

Optionally, the impedance measuring unit is also a unit as described herein.

<u>BRIEF DESCRIPTION OF THE DRAWINGS</u>

Exemplary embodiments of the invention are described in the following sections with reference to the drawings. The drawings are not necessarily to scale and the same reference numbers are generally used for the same or related features that are shown on different drawings.

Figs. 1A, 1B and 1C show schematic views, respectively from the side, the back, and the face, of a unit combining IPG electrodes and a PPG sensor, according to an exemplary embodiment of the invention;

Fig. 1D is a schematic view of IPG electrodes according to another exemplary embodiment of the invention;

Fig. 2 is a schematic perspective view showing placement on the temples of the units shown in Figs. 1A-1C, according to an exemplary embodiment of the invention;

Fig. 3 is a schematic cut-away view of the head with the units placed on it as in Fig. 2, showing current paths through the scalp and through the brain, produced by the IPG electrodes;

Fig. 4 shows a schematic plot of IPG and PPG signals as a function of time, generated by the units placed on the head as in Fig. 2;

Fig. 5 shows a schematic plot of the variation in cerebral blood volume as a function of time during two cardiac cycles, derived by taking a difference between the IPG signal and the PPG signal shown in Fig. 4;

Fig. 6 shows a schematic plot of IPG and PPG signals as a function of time, similar to the signals shown in Fig. 4, but extending over a longer time interval and measured while the subject is hyperventilating;

Fig. 7 shows a schematic plot of an IPG signal as a function of time, illustrating a method of estimating changes in cerebral blood flow according to an exemplary embodiment of the invention;

Fig. 8 shows a schematic plot of an IPG signal and its time derivative as a function of time, illustrating a method of estimating changes in cerebral blood flow according to another exemplary embodiment of the invention; and

DETAILED DESCRIPTION OF EXEMPLARY EMBODIMENT

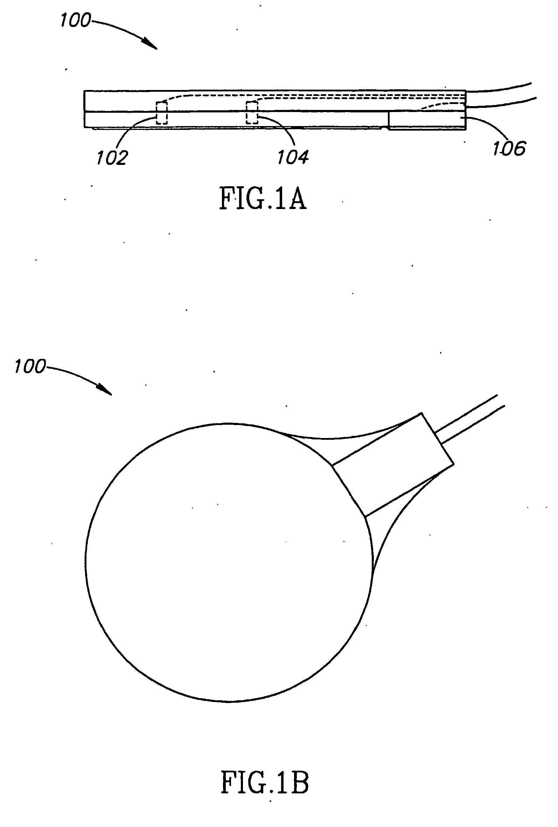

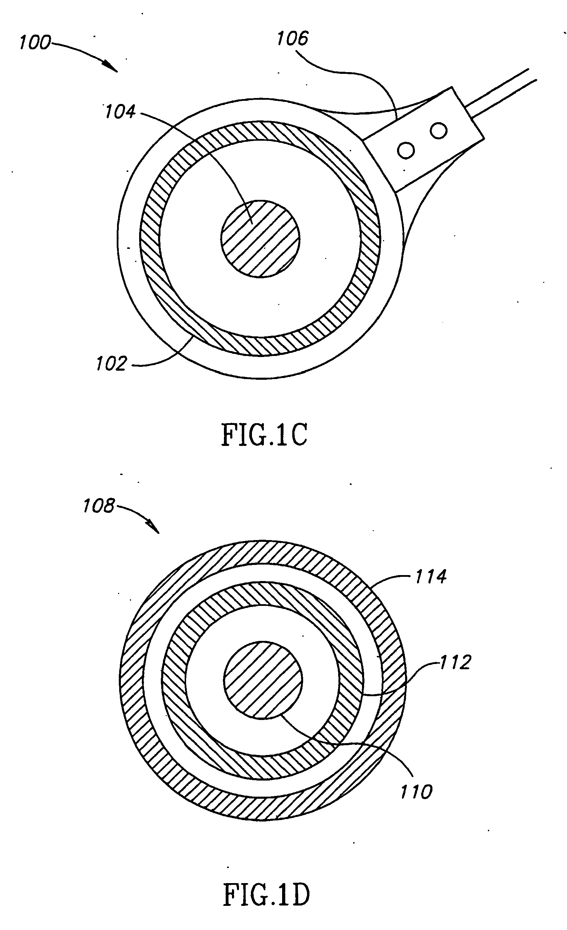

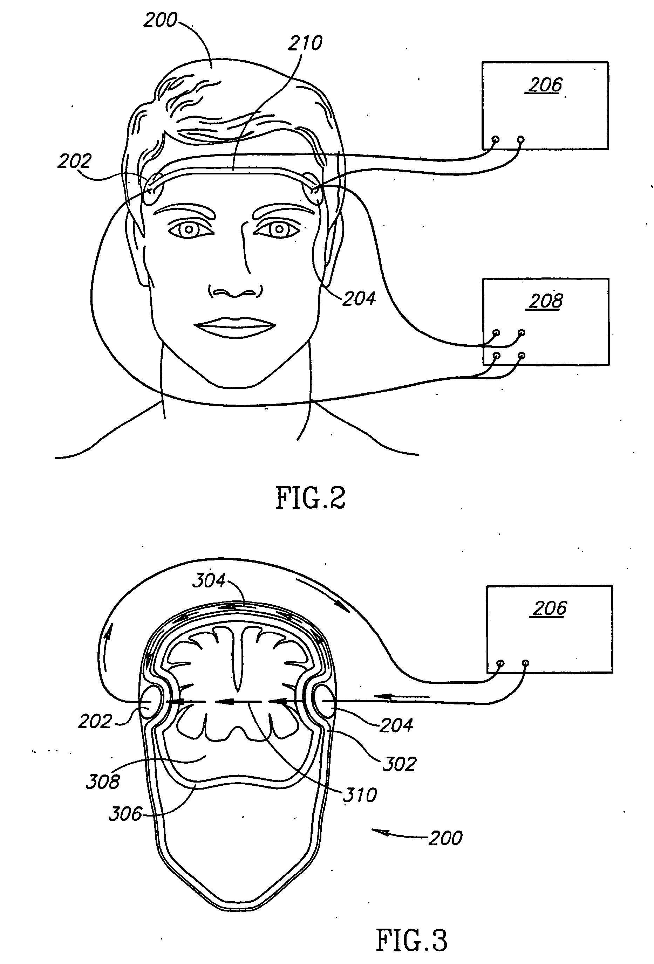

Figs. 1A, 1B, and 1C respectively show side, back, and face views of a unit 100 which optionally combines a current electrode 102 and a voltage electrode 104 for impedance plethysmography (IPG), and a sensor 106 for photoplethysmography (PPG), according to an exemplary embodiment of the invention. The face side of unit 100, shown in Fig. 1C, is the side that is placed against the skin, as shown in Fig. 2. As shown in Fig. 2, two such units, placed for example on opposite sides of the head, are optionally used for IPG, passing current from one unit to the other and measuring the voltage between them. For reasons described below, alternating current is generally used.

PPG sensor 106 measures the color of the skin to determine a degree of perfusion of oxygenated blood in the skin adjacent to unit 100, as described, for example, by J. Webster, "Measurement of Flow and Volume of Blood," in John G. Webster (ed.), Medical Instrumentation: Application and Design (Wiley, 1997), the disclosure of which is incorporated herein by reference. Optionally, PPG sensor 106 incorporates a digital signal processor which converts the raw sensor signal into a usable output signal. Optionally, unit 100 also includes a digital signal processor which processes voltage and/or current and/or photo reflection data of the electrodes and/or PPG in one or both units. Alternatively, the raw signal from sensor 106 and/or data from the electrodes is processed partly or entirely by an external signal processor not located in unit 100.

Alternatively, instead of having separate current and voltage electrodes, unit 100 has a single electrode, used both for carrying current and for measuring voltage. However, using separate electrodes for carrying current and measuring voltage has the potential advantage that the measured voltage may not be very sensitive to a high contact resistance between the electrodes and the skin, or to a high resistance across the epidermis, one or both of which can dominate the voltage drop between the current electrodes on opposite sides of the head. The contact resistance and the epidermis resistance have little or no dependence on blood flow, so it is generally desirable for the IPG signal not to be sensitive to the contact and epidermis resistance. This goal is optionally achieved by using an annular shape for current-electrode 102, and locating voltage-electrode 104 in the center of the annulus, but substantially electrically decoupled from it. The radial thickness of the annulus of electrode 102, and the gap between electrodes 102 and 104, are optionally at least somewhat greater than the thickness of the epidermis under the electrodes, for example at least twice as great. Optionally, the radial thickness of the annulus of electrode 102 is at least 2 mm, or at least 5 mm, or at least 1 cm. Optionally, the gap between electrodes 102 and 104 is at least 2 mm, or at least 5 mm, or at least 1 cm, or intermediate or smaller values.

With this geometry of electrodes 102 and 104, and with current-electrode 102 making good contact with the skin over the surface of the electrode, the current across the epidermis will be broadly distributed, compared to the thickness of the epidermis. The high electric field in the high resistivity epidermis will be largely confined to the region under the current electrode, with a much lower fringing field reaching voltage measuring electrode 104. But the potential in the much lower resistivity dermis will be fairly uniform under unit 100, and the potential of electrode 104 will be close to this potential. The same will be true under the unit on the other side of the head. The voltage difference between the two voltage electrodes 104 on the two sides of the head will be close to the difference in potential in the dermis under the two electrodes. For a given current, this potential difference depends on the impedance of the dermis of the temples and the scalp, and the impedance of the cranium and the brain, as described below in connection with Fig. 3, rather than on the impedance across the epidermis.

An alternative configuration 108 for the voltage and current electrodes is shown in Fig. 1D. Current is injected through electrode 110, located in the center, and voltage is measured at electrode 112, in the form of an annulus surrounding electrode 110, which is electrically well isolated from electrode 110. An additional electrode 114, also in the form of an annulus, surrounds electrode 112, and injects whatever current is necessary in order to remain at the same voltage as electrode 110. However, optionally, only the current injected through electrode 110 is considered for purposes of finding the impedance. With configuration 108, there will be very little radial electric field, and hence very little radial current flow, in the dermis under the region between electrodes 110 and 114. Hence, the current from electrode 110 will be directed mostly into the head, and relatively more of this current will flow through the brain as opposed to flowing through the scalp, while most of the current flowing through the scalp will be injected by electrode 114, and may be ignored for purposes of measuring the impedance. With this configuration, the impedance measurement will be more sensitive to the impedance of the brain, and less sensitive to the impedance of the scalp. Optionally, the thicknesses of electrodes 112 and 114, and the gaps between them and between electrodes 110 and 112, have the same possible dimensions as those mentioned above for electrodes 102 and 104.

Alternatively or additionally, the current through electrode 114 is also measured, and compared to the current through electrode 110, in order to estimate the ratio of the scalp path impedance to the cerebral path impedance. This ratio may be used to find a weighting factor to be used for the PPG signal when subtracting the PPG signal from the IPG signal, instead of or in addition to the methods described above for finding the weighting factor.

Alternatively, instead of the electrode configurations shown in Figs. 1C and 1D, any of the electrode configurations described in US patent application 10/893,570 is used, or any other electrode configuration is used in which the current electrode is adjacent to the voltage electrode. If the current electrode has dimensions that are large compared to the thickness of the epidermis, and the voltage electrode is separated from the current electrode by a similar distance, then the voltage electrode will measure a potential that tends to be close to the potential at the dermis under the voltage and current electrodes, largely excluding the voltage drop across the epidermis.

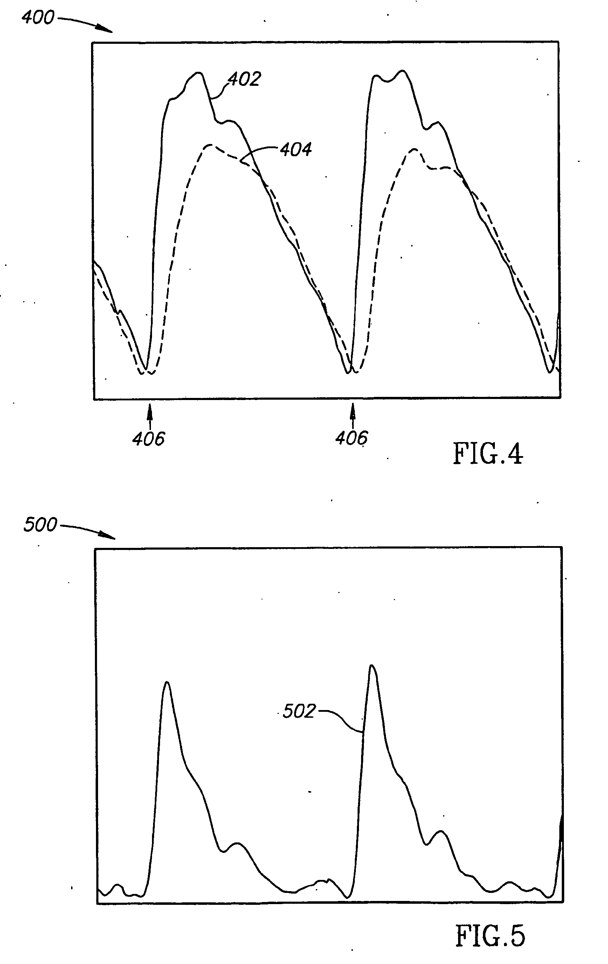

Fig. 2 shows a head 200 with units 202 and 204 placed on the temples on each side of the head, according to an exemplary embodiment of the invention. Optionally, each of units 202 and 204 is like unit 100 in Figs. 1A-1C, including both IPG electrodes and PPG sensors. A power supply 206 passes current between the current-electrodes in units 202 and 204, and a voltage difference is measured between the voltage-electrodes in units 202 and 204, while PPG data is optionally supplied by the PPG sensors in both units. Alternatively, only one of units 202 and 204 has a PPG sensor combined with it, or only one of the PPG sensors is used, or neither unit has a PPG sensor combined with it and a separate PPG sensor is used. A data analyzer 208 uses the voltage difference between the voltage electrodes, together with the PPG data, to estimate the cerebral blood flow, as will be described below in the description of Figs. 4 and 5.

Optionally, a C-shaped spring device 210 connects units 202 and 204, and provides a force to keep units 202 and 204 in place on the temples, similar to headphones. Alternatively, suction cups, such as those used for electrocardiographs, are used to keep units 202 and 204 in place on the temples, or any other method known in the art, for example an adhesive, is used to keep units 202 and 204 in place on the temples.

Alternatively, instead of placing units 202 and 204 on the temples, they are placed at other locations on the head, for example on the forehead and in the back of the head. Although the two electrodes need not be placed on opposite sides of the head, placing them on at least approximately opposite sides of the head has the potential advantage that relatively more current goes through the interior of the skull, rather than through the scalp. Placing the electrodes on the temples has the potential advantage that there is no need to shave the skin before placing the electrodes, and the skull is relatively thin at the temples, also causing relatively more of the current to go through the brain rather than through the scalp. Placing an electrode over one of the closed eyelids, or over the foramen magnum at the base of the skull, or over the ears or inside the ear canal (using an electrode design which fits into the ear canal, such as that shown in PCT application WO 03/059164) also allows current to get into the interior of the skull relatively efficiently.

In some embodiments of the invention, there are more than two such units placed on the head, and, for example, current is passed between different pairs of units while the voltage difference is measured between different pairs of units, not necessarily the same units that current is being passed between. Such an arrangement, using impedance imaging algorithms, can provide additional information about the impedance distribution inside the head, but the data analysis is more complicated than with only two electrodes, and the electrodes take longer to place.

For safety reasons, the units generally use alternating current, for example in the frequency range of a few kilohertz to several tens of kilohertz. Frequencies above about 100 kHz may give impedance data that is less sensitive to blood flow than lower frequencies, since above about 100 kHz the currents can easily flow through the cell membranes, which act like capacitors, and across the interiors of the cells. At frequencies well below 100 kHz, the currents are largely confined to the extra-cellular fluid, and the impedance tends to be more sensitive to blood volume.

Fig. 3 shows a cut-away view of head 200, seen from the front, with units 202 and 204 on the two temples, as in Fig. 2. A cross-sectional cut has been made most of the way through the head in Fig. 3, but in order to show the location of units 202 and 204 on the temples, the skin and skull of the temples have been left in place, in front of the cross-sectional cut. Current between the current electrodes in units 202 and 204 can travel on different paths. Scalp 302 has a relatively low resistivity beneath the epidermis, and a large part of the current travels through the scalp, on path 304, going around skull 306, which has a higher resistivity. Interior 308 of the skull, including the brain and associated blood vessels, also has a relatively low resistivity. Particularly if the current electrodes are fairly wide, a significant part of the current goes through the skull and across the brain, on path 310, since the part of path 310 that goes through the high resistivity skull is relatively short and has wide cross-section, while path 304 through the lower resistivity scalp is much longer and has a much smaller cross-section. If configuration 108 shown in Fig. 1D is used, then a relatively larger part of the current from electrode 110 will tend to go on path 310, through the brain, while a relatively larger part of the current from electrode 114 will tend to go on path 304, through the scalp.

To illustrate how the IPG signal can depend on cerebral blood volume and on scalp blood volume, we note that the impedance $R$ (the ratio of voltage to current) between units 202 and 204 may be expressed as

$$R = \frac{R_S R_B}{R_S + R_B}$$

where $R_B$ is the impedance along path 310 through the skull and brain, and $R_S$ is the impedance along path 304 through the scalp, which is parallel to path 310. Each of these impedances has a constant part which is independent of the phase of the cardiac cycle, and a much smaller part which varies with the phase of the cardiac cycle, due to the change in blood volume in the brain and in the scalp. Thus,

$$R_B = R_{B0} + \Delta R_B$$

$$R_S = R_{S0} + \Delta R_S$$

Then the impedance between units 202 and 204 may be expressed as $R = R_0 + \Delta R$, where $\Delta R$, the small time varying part of the impedance, is given by

$$\Delta R = \frac{R_S}{R_S + R_B} \Delta R_B + \frac{R_B}{R_S + R_B} \Delta R_S$$

to first order in $\Delta R_B$ and $\Delta R_S$. It should be noted that these impedances are mostly resistive at the frequencies typically used, well below 100 kHz, and this is especially true for the variations in the impedances over a cardiac cycle, since they depend on the volume of blood, which is located outside the cell membranes. Higher resistance is associated with a lower volume of blood, so - $\Delta R_B$ and - $\Delta R_S$ are measures respectively of change in cerebral blood volume, and change in blood volume in the scalp. The PPG signal also measures change in blood volume in the scalp, and is approximately a linear function of - $\Delta R_S$ since the signals are small. By subtracting an appropriately weighted PPG signal, proportional to - $\Delta R_S$ , from the IPG signal - $\Delta R$ , we obtain a signal proportional to - $\Delta R_B$, and hence a linear function of the time-varying part of the cerebral blood volume. One or both of the change in cerebral blood volume over each cardiac cycle and/or the maximum of the time derivative of the cerebral blood volume, are optionally used as an indication of the relative cerebral blood flow.

The cerebral blood volume varies during a cardiac cycle because the arterial blood flow into the brain is pulsatile, while the venous blood flow out of the brain is approximately uniform in time. There is some blood flow into the brain even at the time of diastolic pressure, and this baseline cerebral blood flow cannot be determined directly by measuring changes in cerebral blood volume. However, since the time-varying component is a significant fraction of the total cerebral blood flow, measuring the change in cerebral blood volume during a cardiac cycle may provide a clinically useful relative measure of cerebral blood flow.

Fig. 4 shows an exemplary plot 400 of the IPG signal - $\Delta R$, labeled 402, shown as a solid curve, and a weighted PPG signal 404, shown as a dashed curve, as a function of time. Signals 402 and 404 are both plotted in arbitrary units, and alternatively signal 404 could be considered the original PPG signal and signal 402 could be weighted, or both signals could be weighted, since only their ratio matters in plot 400. An R-wave, from an electrocardiogram, has peaks at times 406. Note that shortly after the peak of each R-wave, IPG signal 402 and PPG signal 404 both start to rise, as blood flows into the brain and into the scalp, but the rise in the IPG signal starts earlier, and is much more rapid initially, than the rise in the PPG signal. This is believed to be due to the fact that the arteries supplying blood to the brain have a larger diameter, and lower hydrodynamic resistance to blood flow, than the small arteries supplying blood to the scalp. Later in each cardiac cycle, when the blood has had time to flow into the scalp, we expect the IPG signal to be dominated by the blood volume in the scalp. Hence, the weighting factor for PPG signal 404 has optionally been chosen so that weighted PPG signal 404 is approximately equal to IPG signal 402 during an interval late in each cardiac cycle, for example during the last third of each cardiac cycle, when the blood pressure and signals 402 and 404 are falling, before the next peak of the R-wave.

Alternatively, the weighting factor is chosen by other methods which evaluate, at least approximately, the ratio of current through the cranium to current through the scalp.

In some embodiments of the invention, the weighting factor is set equal to the square root of the ratio of the power spectrum of the IPG signal, integrated over a range of frequencies, to the power spectrum of the PPG signal, integrated over the same range. Optionally, the range of frequencies is a range within which the PPG signal is similar to the IPG signal, as indicated, for example, by a high cross-power spectrum between the IPG and PPG signals. For example, the range of frequencies is centered at the peak of the cross-power spectrum, and extends to each side of the peak by an amount equal to or proportional to the rms width of the peak of the cross-power spectrum. Alternatively, the range of frequencies is defined to include all frequencies for which the cross-power spectrum is greater than a certain fraction (for example, half) of the geometric mean of the magnitudes of the IPG and PPG power spectra. Optionally, the two power spectra are weighted within the range of frequencies, for example according to the value of the cross-power spectrum. In this case, the integration over frequency need not be over a limited range of frequencies.

Fig. 5 shows a plot 500 of a signal 502 equal to the difference between IPG signal 402 and weighted PPG signal 404, as a function of time.

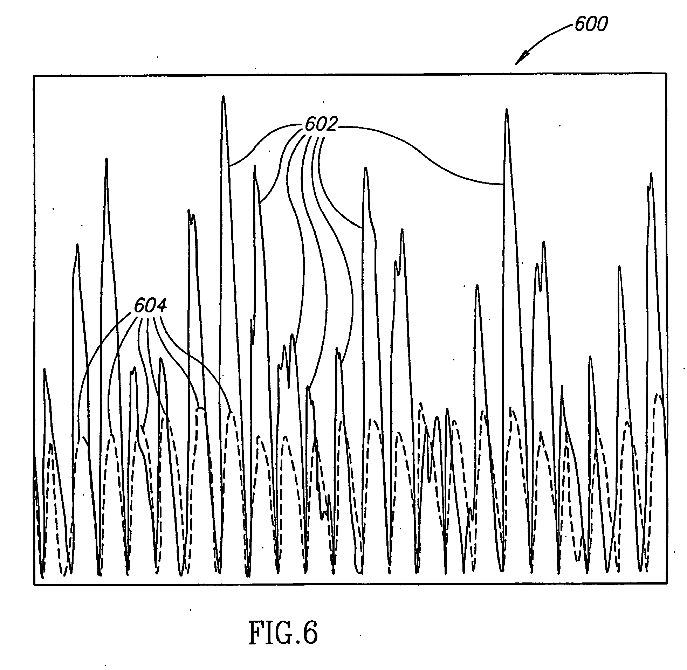

In some embodiments described, the cerebral blood volume is estimated from the IPG signal alone but these embodiments do not form part of the invention . This may be justified because there is evidence that early in each cardiac cycle, and even up to the peak in the IPG signal, the time-dependent part of the IPG signal is largely dominated by changes in cerebral blood volume. For example, Fig. 6 shows a plot 600 of an IPG signal 602, plotted as a solid line, and a PPG signal 604, plotted as a dashed line, measured while the subject was voluntarily hyperventilating. The hyperventilation produces large fluctuations in the peak value of the IPG signal from one cardiac cycle to another, and much smaller fluctuations in the peak value of the PPG signal from one cardiac cycle to another. Since the time dependence of the PPG signal is believed to be due almost entirely to changes in the scalp blood volume, the fact that the IPG signal behaves very differently from the PPG signal indicates that the IPG signal is not dominated by the changes in scalp blood volume, but by something else, presumably changes in cerebral blood volume. One method of estimating the time varying part of the cerebral blood volume is just to assume that the change in cerebral blood volume is proportional to the peak value of the IPG signal for each cardiac cycle.

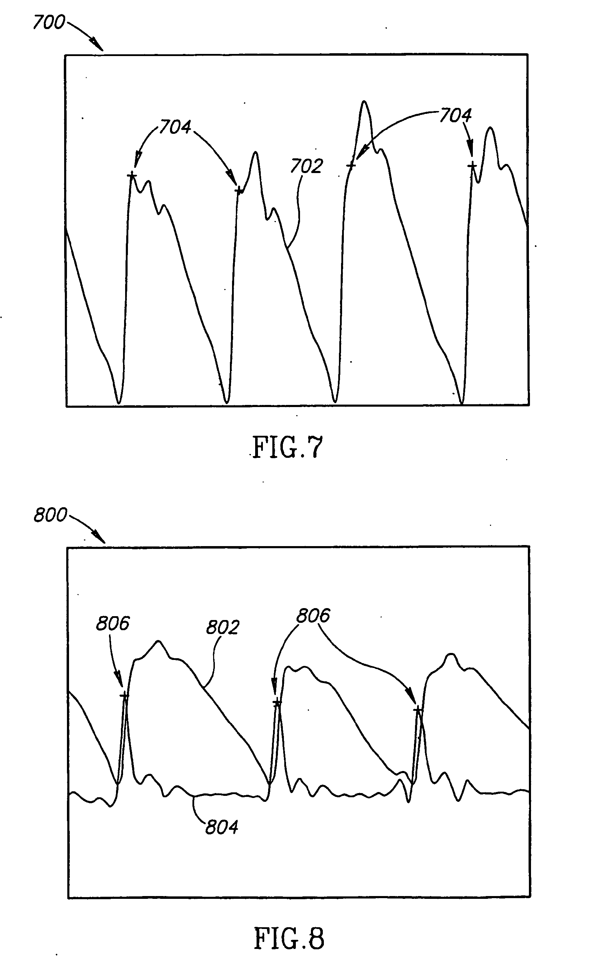

Fig. 7 illustrates another method not forming part of the invention of estimating the changes in cerebral blood volume, again using only the IPG signal. Plot 700 shows an IPG signal 702 as a function of time, for four cardiac cycles. In each cardiac cycle, the value of the IPG signal is measured at its first local peak following the minimum (or following the peak in the R-wave, which occurs at about the same time as the minimum in the IPG signal). Optionally, if there is an inflection point in the IPG signal before the first local peak, then the value of the IPG signal is measured at the inflection point. This is true, for example, in the third cardiac cycle shown in plot 700. These values of the IPG signal for each cardiac cycle are indicated by small crosses 704 in plot 700. Using these values of the IPG signal in each cardiac cycle may better reflect the change in cerebral blood volume than using the peak IPG signal in each cardiac cycle. This may be true, for example, because these values occur earlier in each cardiac cycle, when the IPG signal is more dominated by the time-dependent part of the cerebral blood volume, and is less sensitive to the scalp blood volume.

Fig. 8 illustrates yet another method not forming part of the invention of estimating the changes in cerebral blood volume, using only the IPG signal. Plot 800 shows an IPG signal 802 as a function of time, for three cardiac cycles, and a signal 804 proportional to the time derivative of IPG signal 802. The peak of signal 804, i.e. the peak rate of rise of IPG signal 802, is measured in each cardiac cycle, and indicated by small crosses 806 in plot 800. To the extent that the peak in signal 804 occurs early enough in each cardiac cycle that IPG signal 802 is still dominated largely by changes in cerebral blood volume rather than by changes in scalp volume, the peak value of signal 804 may be a good indication of the change in cerebral blood volume during that cardiac cycle, perhaps a better indication than the peak value of the IPG signal.

In any of the methods illustrated in Figs. 6-8, the PPG signal is optionally recorded as well, for example to verify that the scalp blood volume is not changing very much early in each cardiac cycle, at the times when the IPG signal is used to estimate the change in cerebral blood volume. In some embodiments of the invention, two or more of the methods illustrated in Figs. 5-8 are used to estimate the change in cerebral blood volume, for example by taking a weighted average of the change in cerebral blood volume determined by each method. Different methods might work best for different patients who have different medical conditions. For example, if a patient is suffering from a condition in which cerebral blood flow is likely to be reduced more than scalp blood flow, then the changes in scalp blood flow may dominate the IPG signal even early in the cardiac cycle, and it may be best to use the method illustrated in Fig. 5, which makes use of both the IPG signal and the PPG signal. In a case where cerebral blood flow and scalp blood flow are likely to be reduced at the same time, for example in the case of a patient undergoing cardiac surgery, it may be better or easier to use one of the methods that depends only on the IPG signal.

The invention has been described in the context of the best mode for carrying it out. It should be understood that not all features shown in the drawings or described in the associated text may be present in an actual device, in accordance with some embodiments of the invention. Furthermore, variations on the method and apparatus shown are included within the scope of the invention, which is limited only by the claims. In addition, while the invention has been described in some cases mainly as method, the scope of the invention also includes apparatus programmed to perform the method, for example, dedicated circuitry, hardware, firmware and/or software, including computer-readable media with suitable software thereon. Also, features of one embodiment may be provided in conjunction with features of a different embodiment of the invention. As used herein, the terms "have", "include" and "comprise" or their conjugates mean "including but not limited to."

**Claims**

1. A method of estimating cerebral blood flow, comprising:

   a) obtaining a measure (402) of time-varying blood volume in the head, using impedance plethysmography;
   b) obtaining a measure (404) of time-varying blood volume in the scalp; and
   c) using the measure of time-varying blood volume in the head and time-varying blood volume in the scalp to estimate the cerebral blood flow (502).

2. A method according to claim 1, wherein obtaining a measure of time-varying blood volume in the scalp comprises using photoplethysmography.

3. A method according to claim 1 or claim 2, wherein estimating the cerebral blood flow comprises estimating the relative cerebral blood flow as it changes over time.

4. A method according to any of the preceding claims, wherein using the measures of time-varying blood volume comprises finding a difference between weighted measures of time-varying blood volume.

5. A method according to claim 4, wherein the measures of time-varying blood volume are weighted to have at least approximately the same value at a time in the cardiac cycle when the blood pressure is falling.

6. A method according to claim 4, wherein the measures of time-varying blood volume are weighted to have approximately equal power spectra at frequencies for which the cross-power spectrum between the measures of time-varying blood volume is relatively high.

7. A method according to any of the preceding claims, wherein obtaining a measure of blood volume in the head using impedance plethysmography comprises:

   a) passing a current through the head (200) using two current-carrying electrodes (102); and
   b) measuring a voltage across the head, associated with the current, using two voltage-measuring electrodes (104).

8. A method according to claim 7, also including applying to the head an annular electrode (114) surrounding at least one of the current-carrying electrodes (110), and maintaining the annular electrode at a same voltage as the current-carrying electrode it surrounds, thereby suppressing radial current from said current-carrying electrode.

9. A method according to claim 7 or claim 8, wherein the voltage-measuring electrodes (104 or 112) are distinct from, and substantially electrically decoupled from, the current-carrying electrodes (102 or 110).

10. A method according to any of claims 7-9, wherein obtaining a measure of blood volume in the head (200) using impedance plethysmography comprises placing the two cuitent-carrying electrodes on the left (204) and right (202) temples respectively.

11. A method according to any of claims 7-10, wherein obtaining a measure of blood volume in the head using impedance plethysmography comprises placing each of the two voltage-measuring electrodes on the head in a position adjacent to a different one of the current-canying electrodes.

12. A method according to claim 11, wherein obtaining a measure of blood volume in the scalp using photoplethysmography comprises placing a photoplethysmography sensor (106) on the head adjacent to one of the current-carrying electrodes (102) and to the voltage-measuring electrode (104) which is adjacent to said current-carrying electrode.

13. A method according to any of the preceding claims, wherein obtaining a measure of time-varying blood volume in the scalp comprises using surface impedance plethysmography.

14. A system for estimating cerebral blood flow, comprising:

   a) a power supply (206);
   b) two electrode units (100, or 108, or 202 and 204), each comprising a current-carrying electrode (102) adapted to pass current from the power supply through the head (200) when the units are placed on the head on different sides, and a voltage-measuring electrode (104), the same as or different from the current-carrying electrode, adopted to measure voltage across the head when the current-carrying electrodes are passing current through the head;
   c) a plethysmography sensor (106) adapted for measuring blood flow in a scalp; and
   d) a data analyzer (208) which calculates a cerebral blood flow using impedance data obtained from the voltage across the head measured by the voltage-measuring electrodes, and from data on scalp blood flow generated by plethysmography sensor.

15. A system according to claim 14, wherein said plethysmography sensor adapted for measuring blood flow in a scalp comprises a photoplethysmography sensor.

16. A system according to claim 15, also comprising a signal processor configured to process one or both of data from the photoplethysmography sensor and the impedance data.

17. A system according to claim 15 or claim 16, wherein, for at least one of the electrode units, the voltage-measuring electrode is different from the current-carrying electrode.

18. A system according to claim 17, wherein the current-carrying and voltage-measuring electrodes are configured such that the voltage-measuring electrode will measure a potential substantially equal to a potential at the dermis, largely excluding the voltage drop across the epidermis, when the current-carrying electrodes are passing current through the head.

19. A system according to claim 14-18, wherein said plethysmography sensor adapted for measuring blood flow in a scalp comprises a surface impedance plethysmography sensor.

20. A system according to any of claims 14-19, wherein the plethysmography sensor (106) adapted for measuring blood flow in a scalp is combined with one of the electrode units (100).

21. A system according to claim 20, wherein the other electrode unit comprises a second plethysmography sensor adapted for measuring blood flow in a scalp.

22. A system according to any of claims 14-21 for estimating cerebral blood flow, adapted for use in patients of a range of degree of maturity, wherein, for at least one of the electrode units, the current-carrying electrode comprises an annulus surrounding the voltage-measuring electrode, and the radial thickness of the annulus and the gap between the current-carrying and voltage-measuring electrodes are each at least twice as great as a typical thickness of the epidermis in patients of said range of degree of maturity.

23. A system according to claim 22, wherein the radial thickness of the annulus and the gap between the current-carrying and voltage-measuring electrodes are each at least 1mm.

24. A system according to any of claims 14-23 for estimating cerebral blood flow, comprising an annular electrode (114) surrounding the current-carrying electrode, thereby suppressing radial current from the current-carrying electrode when the annular electrode is maintained at the same voltage as the current-carrying electrode.

**Patentansprüche**

1. Verfahren zum Schätzen der Hirndurchblutung, umfassend:

   a) Erhalten eines Messwerts (402) eines zeitlich veränderlichen Blutvolumens im Kopf unter Verwendung von Impedanzplethysmographie;
   b) Erhalten eines Messwerts (404) eines zeitlich veränderlichen Blutvolumens im Skalp; und
   c) Verwenden des Messwerts eines zeitlich veränderlichen Blutvolumens im Kopf und eines zeitlich veränderlichen Blutvolumens im Skalp zum Schätzen der Hirndurchblutung (502).

2. Verfahren nach Anspruch 1, wobei das Erhalten eines Messwerts eines zeitlich veränderlichen Blutvolumens im Skalp das Verwenden von Photoplethysmographie umfasst.

3. Verfahren nach Anspruch 1 oder 2, wobei das Schätzen der Hirndurchblutung das Schätzen der relativen Hirndurchblutung in der Veränderung über die Zeit umfasst.

4. Verfahren nach einem der vorstehenden Ansprüche, wobei das Verwenden der Messwerte eines zeitlich veränderlichen Blutvolumen das Bestimmen eines Unterschieds zwischen gewichteten Messwerte eines zeitlich veränderlichen Blutvolumens umfasst.

5. Verfahren nach Anspruch 4, wobei die Messwerte eines zeitlich veränderlichen Blutvolumens gewichtet werden, um zumindest ungefähr den gleichen Wert zu einem Zeitpunkt im Herzzyklus zu haben, wenn der Blutdruck fällt.

6. Verfahren nach Anspruch 4, wobei die Messwerte eines zeitlich veränderlichen Blutvolumens gewichtet werden, um ungefähr gleiche Leistungsspektren bei Frequenzen zu erhalten, bei denen das Leistungskreuzspektrum zwischen den Messweuen eines zeitlich veränderlichen Blutvolumens relativ hoch ist.

7. Verfahren nach einem der vorstehenden Ansprüche, wobei das Erhalten eines Messwerts eines zeitlich veränderlichen Blutvolumens unter Verwendung von Impedanzplethysmographie umfasst:

   a) Leiten eines Stroms durch den Kopf (200) unter Verwendung von zwei stromführenden Elektroden (102); und
   b) Messen einer Spannung im Kopf, die mit dem Strom assoziiert ist, unter Verwendung von zwei spannungsmessenden Elektroden (104).

8. Verfahren nach Anspruch 7, ferner umfassend das Anlegen einer ringförmigen Elektrode (114) an den Kopf, die zumindest eine der Strom-führenden Elektroden (110) umgibt, und Halten der ringförmige Elektrode auf einer gleichen Spannung wie die stromführende Elektrode, die sie umgibt, wodurch radialer Strom von der stromführenden Elektrode unterdrückt wird.

9. Verfahren nach Anspruch 7 oder 8, wobei die spannungsmessenden Elektroden (104 oder 112) von den stromführender Elektroden (102 oder 110) unterschiedlich und im Wesentlichen elektrisch entkoppelt sind.

10. Verfahren nach einem der Ansprüche 7 bis 9, wobei das Erhalten eines Messwerts eines Blutvolumens im Kopf (200) unter Verwendung von Impedanzplethysmographie das Platzieren der beiden stromführenden Elektroden an der linken Schläfe (204) bzw. an der rechten Schläfe (202) umfasst.

11. Verfahren nach einem der Ansprüche 7 bis 10, wobei das Erhalten eines Messwerts eines Blutvolumens im Kopf unter Verwendung von Impedanzplethysmographie das Platzieren jeder der beiden spannungsmessenden Elektroden am Kopf in einer Position benachbart einer unterschiedlichen der stromfülwenden Elektroden umfasst.

12. Verfahren nach Anspruch 11, wobei das Erhalten eines Messwerts eines Blutvolumens im Skalp unter Verwendung von Photoplethysmographie das Platzieren eines Photoplethysmographiesensors (106) am Kopf benachbart einer der stromführenden Elektroden (102) und benachbart der spannungsmessenden Elektrode (104), die der stromführenden Elektrode benachbart angeordnet ist, umfasst.

13. Verfahren nach einem der vorstehenden Ansprüche, wobei das Erhalten eines Messwerts eines zeitlich veränderlichen Blutvolumens im Skalp das Verwenden von Oberflächenimpedanzplethysmographie umfasst.

14. System zum Schätzen der Hirndurchblutung, umfassend:

a) eine Stromversorgung (20b);
b) zwei Elektrodeneinheiten (100 oder 108 oder 202 und 204), wobei jede eine stromführende Elektrode (102), die eingerichtet ist, um Strom von der Stromversorgung durch den Kopf (200) zu leiten, wenn die Einheiten an unterschiedlichen Stellen am Kopf platziert sind, und eine spannungsmessende Elektrode (104) umfasst, die der stromführenden Elektrode gleich oder zu dieser unterschiedlich und eingerichtet ist, um Spannung im Kopf zu messen, wenn die stromführenden Elektroden Strom durch den Kopf leiten;
c) einen Plethysmographiesensor (106), der eingerichtet ist, um die Durchblutung in einem Skalp zu messen; und
d) einen Datenanalysator (208), der eine Hirndurchblutung unter Verwendung von Impedanzdaten aus der Spannung im Kopf, die von den spannungsmessenden Elektroden gemessen wurde, und von Daten zur Skalpdurchblutung, die vom Plethysmographiesensor generiert wurden, berechnet.

15. System nach Anspruch 14, wobei der zur Messung der Durchblutung in einem Skalp eingerichtete Plethysrnographiesensor einen Photoplethysrnographiesensor umfasst.

16. System nach Anspruch 15, ferner umfassend einen Signalprozessor, der konfiguriert ist, um eines von oder beides der Daten des Photoplethysmographiesensors und der Impedanzdaten zu verarbeiten.

17. System nach Anspruch 15 oder 16, wobei die spannungsmessende Elektrode sich bei zumindest einer der Elektrodeneinheiten von der stromführenden Elektrode unterscheidet.

18. System nach Anspruch 17, wobei die stromführenden und spannungsmessenden Elektroden derart konfiguriert sind, dass die spannungsmessende Elektrode ein Potenzial im Wesentlichen gleich einem Potenzial an der Dermis misst, wobei der Spannungsabfall auf der Epidermis größtenteils ausgeschlossen wird, wenn die stromführenden Elektroden Strom durch den Kopf leiten.

19. System nach Anspruch 14 bis 18, wobei der zur Messung der Durchblutung in einem Skalp eingerichtete Plethysmographiesensor einen Oberflächenimpedanzplethysmographiesensor umfasst.

20. System nach einem der Ansprüche 14 bis 19, wobei der zur Messung der Durchblutung in einem Skalp eingerichtete Plethysmographiesensor (106) mit einer der Elektrodeneinheiten (100) kombiniert ist.

21. System nach Anspruch 20, wobei die andere Elektrodeneinheit einen zweiten Plethysmographiesensor umfasst, der eingerichtet ist, um die Durchblutung in einem Skalp zu messen.

22. System nach einem der Ansprüche 14 bis 21 zum Schätzen der Hirndurchblutung, das zur Verwendung bei Patienten verschiedensten Alters eingerichtet ist, wobei die stromführende Elektrode bei zumindest einer der Elektrodeneinheiten einen Ring umfasst, der de spannungsmessende Elektrode umgibt, und wobei die radiale Dicke des Rings und des Spalts zwischen den stromführenden und spannungsmessenden Elektroden jeweils zumindest zweimal so groß wie eine typische Dicke der Epidermis bei Patienten dieses verschiedensten Altersbereichs ist.

23. System nach Anspruch 22, wobei die radiale Dicke des Rings und des Spalts zwischen den stromführenden und spannungsmessenden Elektroden zumindest jeweils 1 mm beträgt.

24. System nach einem der Ansprüche 14 bis 23 zum Schätzen der Hirndurchblutung, umfassend eine ringförmige Elektrode (114), die die stromführende Elektrode umgibt, wodurch der radiale Strom von der stromführenden Elektrode unterdrückt wird, wenn die ringförmige Elektrode auf der gleichen Spannung wie die stromführende Elektrode gehalten wird.

## Revendications

1. Procédé d'estimation de la circulation sanguine cérébrale, comprenant :

   a) l'obtention d'une mesure (402) du volume sanguin variant dans le temps dans la tête, en utilisant une pléthysmographie à impédance ;
   b) l'obtention d'une mesure (404) du volume sanguin variant dans le temps dans le cuir chevelu ; et
   c) l'utilisation de la mesure du volume sanguin variant dans le temps dans la tête et du volume sanguin variant dans le temps dans le cuir chevelu pour estimer la circulation sanguine cérébrale (502).

2. Procédé selon la revendication 1, dans lequel l'obtention d'une mesure du volume sanguin variant dans le temps dans le cuir chevelu comprend l'utilisation d'une pléthysmographie optique.

3. Procédé selon la revendication 1 ou la revendication 2, dans lequel l'estimation de la circulation sanguine cérébrale comprend l'estimation de la circulation sanguine cérébrale relative alors qu'elle varie dans le temps.

4. Procédé selon l'une quelconque des revendications précédentes, dans lequel l'utilisation des mesures du volume sanguin variant dans le temps comprend la recherche d'une différence entre des mesures pondérées du volume sanguin variant dans le temps.

5. Procédé selon la revendication 4, dans lequel les mesures du volume sanguin variant dans le temps sont pondérées pour avoir au moins approximativement la même valeur à un instant dans le cycle cardiaque lorsque la pression sanguine diminue.

6. Procédé selon la revendication 4, dans lequel les mesures du volume sanguin variant dans le temps sont pondérées pour avoir approximativement des spectres de puissance identiques aux fréquences pour lesquelles le spectre de puissance entre les mesures du volume sanguin variant dans le temps est relativement élevé.

7. Procédé selon l'une quelconque des revendications précédentes, dans lequel l'obtention d'une mesure du volume sanguin dans la tête en utilisant une pléthysmographie à impédance comprend :

   a) le passage d'un courant à travers la tête (200) en utilisant deux électrodes de transport de courant (102) ; et
   b) la mesure d'une tension à travers la tête, associée au courant, en utilisant deux électrodes de mesure de tension (104).

8. Procédé selon la revendication 7, comprenant également l'application à la tête d'une électrode annulaire (114) entourant au moins l'une des électrodes de transport de courant (110), et le maintien de l'électrode annulaire à une même tension que l'électrode de transport de courant qu'elle entoure, supprimant de ce fait un courant radial provenant de ladite électrode de transport de courant.

9. Procédé selon la revendication 7 ou la revendication 8, dans lequel les électrodes de mesure de tension (104 ou 112) sont distinctes, et sensiblement découplées électriquement, des électrodes de transport de courant (102 ou 110).

10. Procédé selon l'une quelconque des revendications 7 à 9, dans lequel l'obtention d'une mesure du volume sanguin dans la tête (200) en utilisant une pléthysmographie à impédance comprend le placement des deux électrodes de transport de courant sur les tempes gauche (204) et droite (202), respectivement.

11. Procédé selon l'une quelconque des revendications 7 à 10, dans lequel l'obtention d'une mesure du volume sanguin dans la tête en utilisant une pléthysmographie à impédance comprend le placement de chacune des deux électrodes de mesure de tension sur la tête à une position adjacente à une électrode différente parmi les électrodes de transport de courant.

12. Procédé selon la revendication 11, dans lequel l'obtention d'une mesure du volume sanguin dans le cuir chevelu en utilisant une pléthysmographie optique comprend le placement d'un capteur de pléthysmographie optique (106) sur la tête adjacent à l'une des électrodes de transport de courant (102) et à l'électrode de mesure de tension (104) qui est adjacente à ladite électrode de transport de courant.

13. Procédé selon l'une quelconque des revendications précédentes, dans lequel l'obtention d'une mesure du volume sanguin variant dans le temps dans le cuir chevelu comprend l'utilisation d'une pléthysmographie à impédance de surface.

14. Système pour estimer la circulation sanguine cérébrale, comprenant :

   a) une alimentation (206) ;

b) deux unités d'électrodes (100, ou 108, ou 202 et 204), comprenant chacune une électrode de transport de courant (102) conçue pour faire passer un courant de l'alimentation à travers la tête (200) lorsque les unités sont placées sur la tête sur différents côtés, et une électrode de mesure de tension (104), identique ou différente de l'électrode de transport de courant, conçue pour mesurer la tension à travers la tête lorsque les électrodes de transport de courant font passer un courant à travers la tête ;

c) un capteur de pléthysmographie (106) conçu pour mesurer la circulation sanguine dans un cuir chevelu ; et

d) un analyseur de données (208) qui calcule une circulation sanguine cérébrale en utilisant des données d'impédance obtenues à partir de la tension à travers la tête mesurée par les électrodes de mesure de tension, et à partir de données concernant la circulation sanguine du cuir chevelu générées par le capteur de pléthysmographie.

15. Système selon la revendication 14, dans lequel ledit capteur de pléthysmographie conçu pour mesurer la circulation sanguine dans un cuir chevelu comprend un capteur de pléthysmographie optique.

16. Système selon la revendication 15, comprenant également un processeur de signal configuré pour traiter l'une des données provenant du capteur de pléthysmographie optique et des données d'impédance, ou les deux.

17. Système selon la revendication 15 ou la revendication 16, dans lequel, pour au moins l'une des unités d'électrodes, l'électrode de mesure de tension est différente de l'électrode de transport de courant.

18. Système selon la revendication 17, dans lequel les électrodes de transport de courant et de mesure de tension sont configurées de sorte que l'électrode de mesure de tension mesurera un potentiel sensiblement égal à un potentiel du derme, à l'exclusion généralement de la chute de tension à travers l'épiderme, lorsque les électrodes de transport de courant font passer un courant à travers la tête.

19. Système selon les revendications 14 à 18, dans lequel ledit capteur de pléthysmographie conçu pour mesurer la circulation sanguine dans un cuir chevelu comprend un capteur de pléthysmographie à impédance de surface.

20. Système selon l'une quelconque des revendications 14 à 19, dans lequel le capteur de pléthysmographie (106) conçu pour mesurer la circulation sanguine dans un cuir chevelu est combiné avec les unités d'électrodes (100).

21. Système selon la revendication 20, dans lequel l'autre unité d'électrodes comprend un deuxième capteur de pléthysmographie conçu pour mesurer la circulation sanguine dans un cuir chevelu.

22. Système selon l'une quelconque des revendications 14 à 21 pour estimer la circulation sanguine cérébrale, conçu pour une utilisation pour des patients dans une plage de degré de maturité, dans lequel, pour au moins l'une des unités d'électrodes, l'électrode de transport de courant comprend un anneau entourant l'électrode de mesure de tension, et l'épaisseur radiale de l'anneau et l'espace entre les électrodes de transport de courant et de mesure de tension sont chacun égaux à deux fois une épaisseur type de l'épiderme des patients de ladite plage de degré de maturité.

23. Système selon la revendication 22, dans lequel l'épaisseur radiale de l'anneau et l'espace entre les électrodes de transport de courant et de mesure de tension sont égaux chacun à au moins 1 mm.

24. Système selon l'une quelconque des revendications 14 à 23 pour estimer la circulation sanguine cérébrale, comprenant une électrode annulaire (114) entourant l'électrode de transport de courant, supprimant de ce fait un courant radial provenant de l'électrode de transport de courant lorsque l'électrode annulaire est maintenue à la même tension que l'électrode de transport de courant.

100

102          104                          106

FIG.1A

100

FIG.1B

FIG.1C

FIG.1D

FIG.2

FIG.3

400

402

404

406            406

FIG.4

500

502

FIG.5

FIG.6

FIG.7

FIG.8

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10893570 B
- IL 0300042 W
- US 60348278 B
- US 6819950 B, Mills
- US 5694939 A, Cowings
- US 5396893 A, Oberg
- US 6832113 B, Belalcazar
- US 6169914 B, Holland
- US 6413223 B, Yang
- US 20040034294 A1, Kimball
- WO 03059164 A

**Non-patent literature cited in the description**

- **J. H. SEIPEL ; J. E. FLOAM.** *J. Clinical Pharmacology,* 1975, vol. 15, 144-154
- **R. JEVNING ; G. FERNANDO ; A. F. WILSON.** Evaluation of consistency among different electrical impedance indices of relative cerebral blood flow in normal resting individuals. *J. Biomed Eng.,* 1989, vol. 11, 53-56
- Measurement of Flow and Volume of Blood. **J. WEBSTER.** Medical Instrumentation: Application and Design. Wiley, 1997